# EUROPEAN PATENT APPLICATION

(11) **EP 4 534 269 A1**
(43) Date of publication of application: **09.04.2025**
(21) Application number: 22943886.6
(22) Date of filing: 27.05.2022
(51) Int. Cl.: B29C 33/30, B29C 33/34, A61M 37/00, B29L 31/00

(54) **MOLD SUPPORT UNIT FOR MANUFACTURING MICROSTRUCTURE AND MICROSTRUCTURE MANUFACTURING APPARATUS COMPRISING SAME**

(71) Applicant: Cursus Bio, Seoul 06170 (KR)
(72) Inventor: KIM, Yong Hee, Seoul 04763 (KR); FAKHRAEI LAHIJI, Shayan, Seoul 04763 (KR); JOO, Seung Hwan, Seoul 04763 (KR)
(74) Representative: Jung, Minkyu
(86) International application number: PCT/KR2022/007585
(87) International publication number: WO 2023/229079

(57) **Abstract**

A mold support unit is disclosed. The mold support unit supports molds for manufacturing microstructures, and comprises a support plate in which a plurality of support regions on which the molds are placed are formed to be spaced apart from each other, wherein the support regions include a first support region in which an upper surface on which the mold is placed is disposed at a first angle; and a second support region in which an upper surface on which the mold is placed is disposed at a second angle different from the first angle.

## Description

### [Technical Field]

The present invention relates to a mold support unit for manufacturing a microstructure and a microstructure manufacturing apparatus including the same, and more particularly, to a mold support unit for manufacturing a microstructure capable of filling a mold with a composition using centrifugal force, and a microstructure manufacturing apparatus including the same.

### [Background Art]

As a route of administration for delivering drugs to a body, there are oral, injectable, transdermal types, etc. Oral administration is a convenient way to increase a patient's medication compliance, and active ingredients are delivered to the body in the form of capsules, tablets, and syrups. However, the active ingredient may be deactivated due to first-pass metabolism, etc., in the liver, and an absorption rate of biopharmaceuticals is actually relatively low. Therefore, in order to accurately and quickly express the efficacy of drugs and therapeutic agents, etc., these drugs, therapeutic agents, etc., are administered to the body by penetrating through a skin barrier in the injectable type. When the drugs and therapeutic agents are delivered in the injectable type, there is an advantage that the activity of the active ingredient is maintained, but there are disadvantages such as the risk of infection, inaccurate dose administration, phobia, and pain.

To overcome the limitations of the existing oral and injectable routes of administration, various microstructure transdermal drug delivery systems including minimally invasive microneedles have been developed. Microstructures are mainly manufactured in biodegradable (dissolving), solid, coated, and hollow forms. The biodegradable microstructures are transdermal delivery systems that may formulate various substances including polymers and active ingredients (API/cosmetics or pharmaceuticals) in the form of microneedles, and deliver drugs painlessly by dissolving loaded substances in body fluids after insertion into the skin.

A method for manufacturing mold casting is used as a method for manufacturing a microstructure. The method for manufacturing mold casting fills a mold with a composition using centrifugal force or vacuum and then dries the composition.

However, the method using vacuum causes problems in the uniformity of microstructure manufacturing because the composition does not spread throughout the mold or bubbles are generated in the composition due to the vacuum.

In addition, the method using centrifugal force causes the phenomenon that compositions are accurately filled in molds positioned in a rotational radial direction of a rotating device, but are concentrated to one side in molds not positioned in the rotational radial direction. In order to solve these problems, more compositions are loaded into the mold to manufacture the microstructures. As the amount of composition increases, a base part of the microstructure becomes thicker. When the thickness of the base part becomes thicker, the elasticity of a microstructure array decreases, so there is a high possibility that the microneedles are not accurately inserted into the skin, and there is a problem that the loaded medicine is not delivered quantitatively.

A method for manufacturing a microstructure that can overcome the limitations of the existing microstructure manufacturing method, enable mass production, enable quantitative drug loading, and have high manufacturing uniformity is required.

### [Detailed Description of the Invention]

### [Technical Problem]

The present invention provides a mold support unit for manufacturing a microstructure capable of uniformly filling an entire area of a mold with a composition, and a microstructure manufacturing apparatus including the same.

### [Technical Solution]

According to the present invention, a mold support unit supports molds for manufacturing microstructures and includes: a support plate in which a plurality of support regions on which the molds are placed are formed to be spaced apart from each other, in which the support regions include a first support region in which an upper surface on which the mold is placed is disposed at a first angle, and a second support region in which an upper surface on which the mold is placed is disposed at a second angle different from the first angle.

The support regions may further include a third support region whose upper surface on which the mold is placed is disposed at a third angle different from the first angle and the second angle.

The upper surface of the first support region may be disposed parallel to the upper surface of the support plate, the upper surface of the second support region may be disposed to be inclined at the second angle with respect to the upper surface of the support plate, and the upper surface of the third support region may be disposed to be inclined at the third angle with respect to the upper surface of the support plate.

The third support region may be positioned on an opposite side of the second support region with the first support region interposed therebetween, and the upper surface of the second support region and the upper surface of the third support region may be symmetrical with respect to the first support region.

The first support region to the third support region may be sequentially positioned in one direction, and the third angle with respect to the upper surface of the support plate may be larger than the second angle.

The mold support unit may further include a loading plate that is provided with a plurality of openings formed such that the support regions can be individually inserted inward and has a support jaw formed on the inner side surface that defines the opening and on which the mold is placed.

The support regions and the openings may correspond one-to-one.

A plurality of support legs may be formed on a bottom surface of the support plate and extend downward to a predetermined length.

The plurality of support plates may be stacked in a vertical direction, and the support legs may be placed on the upper surface of the support plate positioned at a lower portion thereof.

According to the present invention, a microstructure manufacturing apparatus includes: a rotation unit rotatable around a first rotation axis; and a mold support unit coupled to the rotation unit at a preset distance from the first rotation axis, capable of relative rotation around the rotation unit around a second rotation axis, and supporting a mold for manufacturing a microstructure, in which the mold support unit includes a support plate having a support region formed on which the mold is placed, and the mold support unit rotates around a second rotation axis by centrifugal force of the rotation unit rotating around the first rotation axis so that the support region may face the first rotation axis.

The plurality of mold support units may be disposed in a ring shape based on the first rotation axis and positioned at the same distance from the first rotation axis.

The support plate may include: a first support region in which an upper surface on which the mold is placed is disposed at a first angle; and a second support region in which an upper surface on which the mold is placed is disposed at a second angle different from the first angle.

The upper surface of the first support region may be disposed parallel to the upper surface of the support plate, and the upper surface of the second support region may be disposed to be inclined at the second angle with respect to the upper surface of the support plate.

The upper surface of the first support region may be disposed parallel to the upper surface of the support plate, and the upper surface of the second support region may be disposed to be inclined with respect to the upper surface of the support plate.

The mold support unit may further include a loading plate that is provided with an opening formed such that the support region can be inserted inward and has a support jaw formed on the inner side surface that defines the opening and on which the mold is placed.

### [Advantageous Effects of the Invention]

According to the present invention, when a rotation unit rotates around a first rotation axis, a mold placed on a support region of a support plate may be disposed toward a first rotation axis, and compositions may be spread to each region of the mold at a constant thickness by a centrifugal force of a rotation unit and accurately filled into a needle groove of the mold.

### [Brief Description of Drawings]

FIG. 1 is a diagram illustrating a microstructure manufacturing apparatus according to an exemplary embodiment of the present invention.
FIG. 2 is a diagram illustrating a high-speed rotation state of the microstructure manufacturing apparatus of FIG. 1.
FIG. 3 is a diagram illustrating a microstructure manufacturing apparatus according to another exemplary embodiment of the present invention.
FIG. 4 is a diagram illustrating a mold support unit of FIG. 3.
FIG. 5 is a diagram illustrating a rotation state of the microstructure manufacturing apparatus of FIG. 4.
FIG. 6 is a diagram illustrating a support plate according to another exemplary embodiment of the present invention.
FIG. 7 is a diagram illustrating a microstructure manufactured using a mold support unit according to an exemplary embodiment of FIG. 4.
FIG. 8 is a diagram illustrating a microstructure manufactured using a mold support unit according to Comparative Embodiment.
FIG. 9 is a plan view illustrating a mold support unit according to another exemplary embodiment of the present invention.
FIG. 10 is a cross-sectional view illustrating the mold support unit of FIG. 9.
FIG. 11 is a diagram illustrating the mold support unit according to still another exemplary embodiment of the present invention.
FIG. 12 is an exploded view illustrating the mold support unit of FIG. 11.
FIG. 13 is a diagram illustrating the mold support unit according to still another exemplary embodiment of the present invention.
FIG. 14 is a diagram illustrating an appearance that the plurality of mold support units of FIG. 13 are stacked.
FIG. 15 is a diagram illustrating an appearance that the mold support unit of FIG. 13 rotates 90° around a second rotation axis.
FIG. 16 is a diagram illustrating the mold support unit according to still another exemplary embodiment of the present invention.
FIG. 17 is a diagram illustrating a microstructure manufacturing apparatus according to still another exemplary embodiment of the present invention.
FIG. 18 is a diagram illustrating a microstructure manufacturing apparatus according to still another exemplary embodiment of the present invention.

### [Best Mode for Carrying out the Invention]

Hereinafter, preferred exemplary embodiments of the present invention will be described in detail with reference to the accompanying drawings. However, the technical idea of the present invention is not limited to exemplary embodiments described herein, but may be implemented in other forms. On the contrary, exemplary embodiments introduced herein are provided to make disclosed contents thorough and complete and sufficiently transfer the spirit of the present invention to those skilled in the art.

In this specification, when an element is referred to as being on another element, it means that an element may be formed directly on another element or that a third element may be interposed between them. In addition, in the drawings, the thickness of layers and regions is exaggerated for efficient description of technical contents.

Also, although terms such as first, second, third, etc., have been used in various exemplary embodiments of this specification to describe various components, these components should not be limited by these terms. These terms are only used to distinguish one component from another component. Therefore, what is referred to as a first component in one exemplary embodiment may also be referred to as a second component in another exemplary embodiment. Each exemplary embodiment described and illustrated herein also includes its complementary exemplary embodiments. In the present specification, the term 'and/or' is used as the meaning including at least one of components arranged before and after the term.

In this specification, singular forms are intended to include plural forms unless the context clearly indicates otherwise. In addition, it should be further understood that the terms "include" or "have" specify the presence of features, numerals, steps, components mentioned in the present specification, or combinations thereof, but do not preclude the presence or addition of one or more other features, numerals, steps, components, or combinations thereof. In addition, in the present specification, "connection" is used to mean both indirectly connecting a plurality of components, and directly connecting them.

In addition, when it is determined that the detailed description of the known functions or configurations in describing the present invention may obscure the gist of the present invention, the detailed description thereof will be omitted.

A microstructure manufacturing apparatus according to various exemplary embodiments described below may manufacture a microstructure capable of delivering a drug to a body. The microstructure is a structure in which a thin base layer and a plurality of needles formed on one surface of the base layer are coupled, and the needles may be inserted into a skin tissue to deliver a drug. The microstructure is manufactured by filling a needle groove (hereinafter referred to as "mold") of a mold for manufacturing a microstructure with a composition. The composition may be a biocompatible or biodegradable material. A biocompatible or biodegradable material is a material that is substantially non-toxic to a human body, chemically inactive, and non-immunogenic, and has the advantage of being dissolved after finally penetrating into the body.

The types of these biocompatible materials are not particularly limited, and for example, hyaluronic acid, polyester, polyhydroxyalkanoates (PHAs), poly(α-hydroxyacids), poly(β-hydroxyacids), poly(3-hydroxybutyrate-co-valerate; PHBV), poly(3-hydroxyproprionate; PHP), poly(3-hydroxyhexanoate; PHH), poly(4-hydroxyacids), poly(4-hydroxybutyrate), poly(4-hydroxyvalerate), poly(4-hydroxyhexanoate), poly(esteramide), polycaprolactone, polylactide, polyglycolide, poly(lactide-co-glycolide; PLGA), polydioxanone, polyorthoester, polyetherester, polyanhydrides, poly(glycolic acid-co-trimethylene carbonate), polyphosphoesters, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHA-PEG, ethylene vinyl alcohol copolymer (EVOH), polyurethanes, silicones, polyester, polyolefin, polyisobutylene and ethylene-alphaolefin copolymers, stylene-isobutylene-styrene triblock copolymers, acrylic polymers and copolymers, vinyl halide polymers and copolymers, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatics, polystyrene, polyvinyl ester, polyvinyl acetate, ethylene-methyl methacrylate copolymer, acrylonitrile-styrene copolymer, ABS resin and ethylene-vinyl acetate copolymer, polyamide, alkyd resin, polyoxymethylene, polyimide, polyether, polyacrylate, polymethacrylate, polyacrylic acid-co-maleic acid, chitosan, dextran, cellulose, heparin, alginate, inulin, starch or glycogen may be used, and at least one selected from the group consisting of hyaluronic acid, polyester, polyhydroxyalkanoates (PHAs), poly(α-hydroxyacid), poly(β-hydroxyacid), poly(3-hydroxybutyrate-co-valerate (PHBV), poly(3-hydroxyproprionate (PHP), poly(3-hydroxyhexanoate (PHH), poly(4-hydroxyacid), poly(4-hydroxybutyrate), poly(4-hydroxyvalerate), poly(4-hydroxyhexanoate), poly(esteramide), polycaprolactone, polylactide, polyglycolide, poly(lactide-co-glycolide (PLGA), polydioxanone, polyorthoester,polyetherester, polyanhydride, poly(glycolic acid-co-trimethylene carbonate), polyphosphoester, polyphosphoesterurethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHAPEG, chitosan, dextran, cellulose, heparin, alginate, inulin, starch, and glycogen may be used.

When the microstructure is a solid microneedle loaded with a biocompatible or biodegradable material, a drug may be additionally loaded. The drug refers to a broad concept, and includes not only therapeutic agents for therapeutic purposes in a narrow sense, but also energy, nano-ingredients, cosmetic ingredients (e.g., anti-wrinkle agents, anti-aging agents, and skin whitening agents), cell culture solutions, etc.

Specifically, the therapeutic agents include chemical drugs, protein/peptide drugs, peptide drugs, nucleic acid molecules for gene therapy, etc.

Examples of the therapeutic agents may include anti-inflammatory drugs, analgesics, antiarthritis drugs, antispasmodics, antidepressants, antipsychotics, tranquilizers, anti-anxiety drugs, narcotic antagonists, antiparkinsonian drugs, cholinergic agonists, anticancer drugs, antiangiogenesis inhibitors, immunosuppressants, antiviral drugs, antibiotics, appetite suppressants, analgesics, anticholinergics, antihistamines, antimigraine drugs, hormones, coronary, cerebrovascular or peripheral vasodilators, birth control pills, antithrombotic agents, diuretics, antihypertensives, cardiovascular disease medications, etc.

In particular, the protein/peptide drugs may include hormones, hormone analogues, enzymes, enzyme inhibitors, signaling proteins or parts thereof, antibodies or parts thereof, single-chain antibodies, binding proteins or binding domains thereof, antigens, attachment proteins, structural proteins, regulatory proteins, toxic proteins, cytokines, transcriptional regulators, blood clotting factors, and vaccines, etc. More specifically, the protein/peptide drugs may include insulin, insulin-like growth factor 1 (IGF-1), growth hormone, erythropoietin, granulocyte-colony stimulating factors (G-CSFs), granulocyte/macrophagecolony stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRHII), gonadorelin, goserelin, histrelin, leuprorelin, lypressin, octreotide, oxytocin, pitressin, secretin, sincalide, terlipressin, thymopentin, thymosine α1, triptorelin, bivalirudin, carbetocin, cyclosporine, exedine, lanreotide, luteinizing hormone-releasing hormone (LHRH), nafarelin, parathyroid hormone, pramlintide, enfuvirtide (T-20), thymalfasin, and ziconotide.

The mold may have various shapes. In the drawings of the present invention, the mold is illustrated as a square shape, but is not limited thereto, and may have a circular or polygonal shape. Depending on the shape of the mold, a square, circular, and polygonal base layer may be manufactured.

Hereinafter, a microstructure manufacturing apparatus according to the present invention will be described in detail.

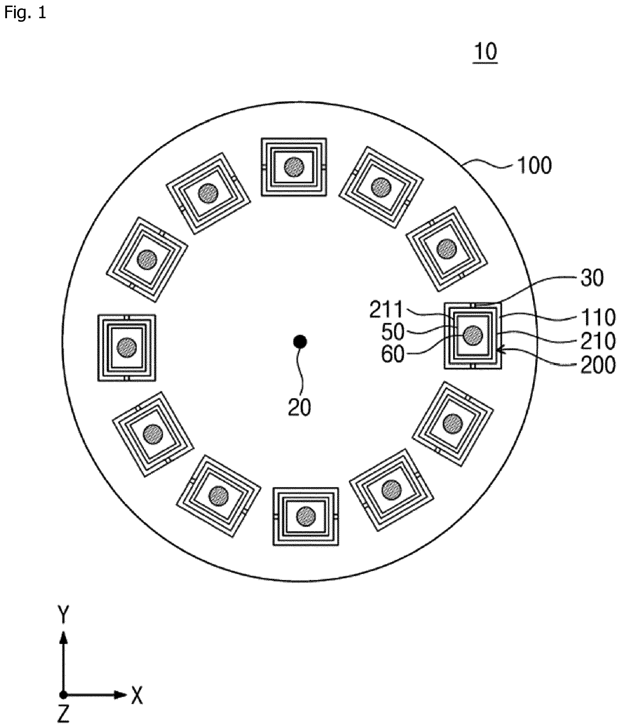

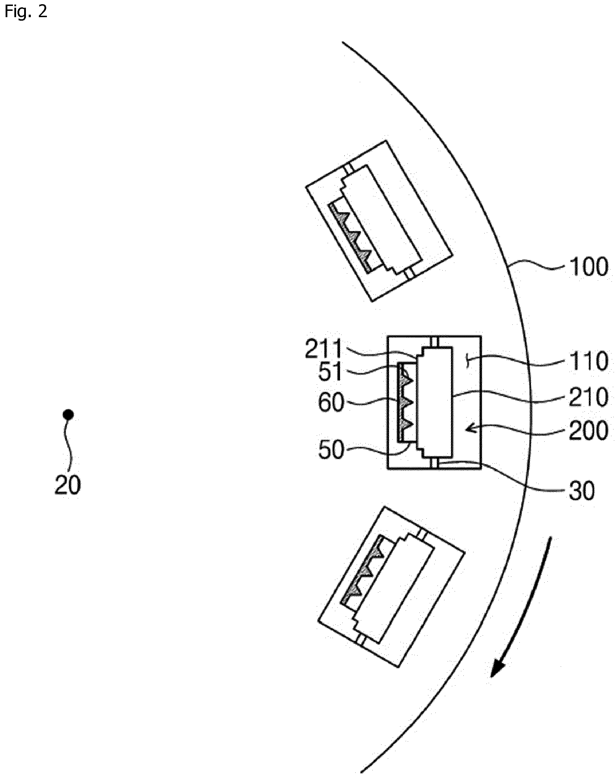

FIG. 1 is a diagram illustrating a microstructure manufacturing apparatus according to an exemplary embodiment of the present invention, and FIG. 2 is a diagram illustrating a high-speed rotation state of the microstructure manufacturing apparatus of FIG. 1.

Referring to FIGS. 1 and 2, a microstructure manufacturing apparatus 10 includes a rotation unit 100 and a mold support unit 200.

The rotation unit 100 is configured to rotate around a first rotation axis 20, and may be provided in various shapes. According to an exemplary embodiment, the rotation unit 100 may be provided as a circular plate rotatable around the first rotation axis 20. According to another exemplary embodiment, the rotation unit 100 may be provided as a circular frame rotatable around the first rotation axis 20. The first rotation axis 20 may be provided perpendicular to the ground.

A receiving space 110 in which the mold support unit 200 may be positioned is formed in the rotation unit 100. According to an exemplary embodiment, the plurality of receiving spaces 110 are formed and disposed in a circular shape around the first rotation axis 20. Each receiving space 110 may be positioned at an equal distance from the first rotation axis 20.

The mold support unit 200 supports the mold 50. The mold support units 200 are each positioned in the receiving spaces 110 and are coupled with the rotation unit 100 to be rotatable around the second rotation axis 30. The second rotation axis 30 is disposed in a tangential direction with respect to the circle, which is the arrangement direction of the mold support units 200. The second rotation axis 30 may be disposed perpendicular to a rotational radial direction of the mold support unit 200.

The mold support unit 200 includes a support plate 210. The support plate 210 has a predetermined area, is a thin plate, and has a support region 211 formed on one side thereof. A mold 50 is placed on the upper surface of the support region 211. The upper surface of the support region 211 is provided as a flat surface.

A composition 60 is supplied to the upper surface of the mold 50 while the mold 50 is placed on the support region 211. The composition 60 may not be directly injected into a needle groove 51 of the mold 50 due to its high viscosity. In this state, when the rotation unit 100 rotates at high speed around the first rotation axis 20, the mold support unit 200 rotates 90° around the second rotation axis 30 by centrifugal force, so that the support plate 210 is disposed perpendicular to the rotational radial direction of the rotation unit 100, and the support region 211 is disposed toward the first rotation axis 20. The centrifugal force of the rotation unit 100 acts perpendicularly to the upper surface of the mold 50 and then spreads radially, so the composition 60 may be uniformly spread into each region of the mold 50 and injected into the needle grooves 51. The composition 60 may penetrate deep into the needle grooves 51 in the direction of the centrifugal force of the rotation unit 100.

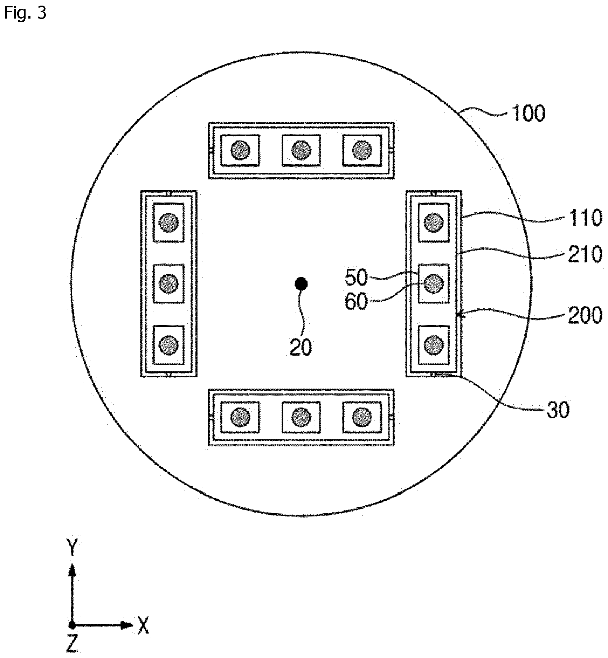

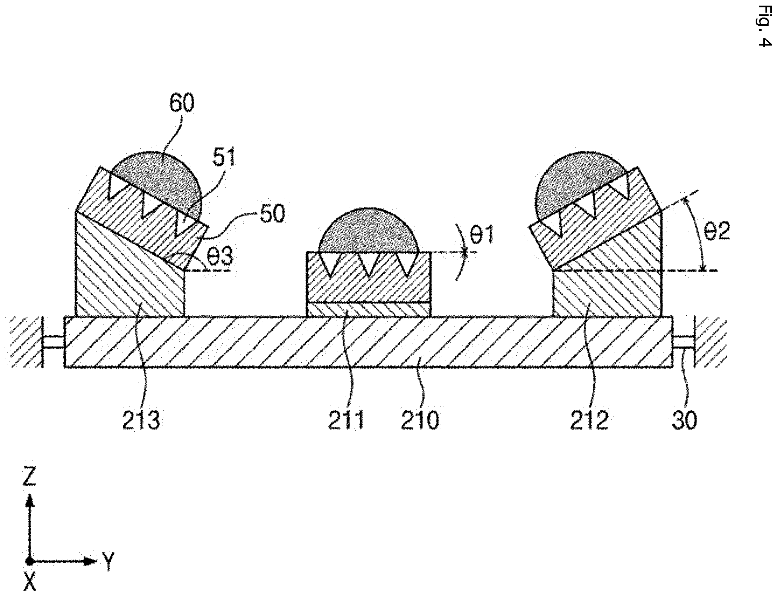

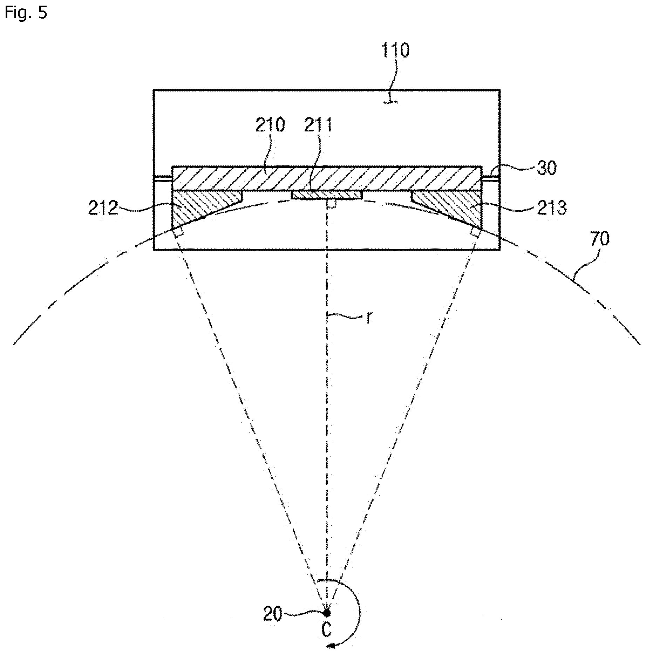

FIG. 3 is a diagram illustrating a microstructure manufacturing apparatus according to another exemplary embodiment of the present invention, FIG. 4 is a diagram illustrating a mold support unit of FIG. 3, and FIG. 5 is a diagram illustrating a rotation state of the microstructure manufacturing apparatus of FIG. 4.

First, referring to FIGS. 3 and 4, the plurality of receiving spaces 110 are formed in the rotation unit 100. According to an exemplary embodiment, four receiving spaces 110 are formed and disposed at 90°. The receiving space 110 is formed to have a predetermined length, and a longitudinal direction thereof is disposed perpendicular to the radial direction of the rotation unit 100.

The mold support units 200 are each positioned in the receiving spaces 110 and are coupled with the rotation unit 100 to be rotatable around the second rotation axis 30. The mold support unit 200 includes the support plate 210. The support plate 210 has an area corresponding to the receiving space 110, and has a plurality of support regions 211, 212, and 213 formed on one surface thereof. The mold 50 is placed in each of the support regions 211, 212, and 213. According to an exemplary embodiment, three support regions 211, 212, and 213 are provided on the upper surface of the support plate 210, the first support region 211 is positioned at the center of the support plate 210, and the second support region 212 and the third support region 213 are positioned on both sides of the first support region 211, respectively.

The upper surface of the first support region 211 is disposed at a first angle θ1. According to an exemplary embodiment, the first angle 211 is an angle that forms 0° with the upper surface of the support plate 210, and the upper surface of the first support region 211 is disposed parallel to the upper surface of the support plate 210.

The upper surface of the second support region 212 is disposed at a second angle θ2. The second angle θ2 is a different angle from the first angle θ1, and may form an angle greater than 0° and smaller than 90° with respect to the upper surface of the support plate 210 based on the center of the support plate 210. According to an exemplary embodiment, the second angle θ2 may form an angle greater than 1° and smaller than 60° with respect to the upper surface of the support plate 210. As a result, the upper surface of the second support region 212 may be disposed to be inclined at the second angle θ2 with respect to the upper surface of the support plate 210. Specifically, the second support region 212 is provided so that a front end adjacent to the first support region 211 is lower in height than the rear end, and the upper surface is provided to be inclined downward at the second angle θ2 toward the first support region 211.

The upper surface of the third support region 213 is disposed at a third angle θ3. The third angle θ3 is a different angle from the first angle θ1 and the second angle θ2, and may form an angle greater than 90° and smaller than 180° with respect to the upper surface of the support plate 210 based on the center of the support plate 210. According to an exemplary embodiment, the third angle θ3 may form an angle greater than 91° and smaller than 150° with respect to the upper surface of the support plate 210. As a result, the upper surface of the third support region 213 may be disposed to be inclined at the third angle θ3 with respect to the upper surface of the support plate 210. Specifically, the third support region 213 is provided so that a front end adjacent to the first support region 211 is lower in height than the rear end, and the upper surface is provided to be inclined downward at the third angle θ3 toward the first support region 211. The upper surface of the third support region 213 may be symmetrical with the upper surface of the second support region 212 with respect to the first support region 211.

Referring to FIG. 5, when the rotation unit 100 rotates at high speed around the first rotation axis 20, the mold support unit 200 rotates 90° around the second rotation axis 30 by centrifugal force, so that one surface of the support plate 210 is disposed perpendicular to the rotational radial direction of the rotation unit 100. In this case, the upper surfaces of the support regions 211, 212, and 213 are disposed in a tangential direction of a virtual circle 70 based on the virtual circle 70 with a radius of a shortest distance r connecting the upper surfaces of each of support regions 211, 212, and 213 in the first rotation axis 20. Therefore, the centrifugal force of the rotation unit 100 is provided perpendicularly to the upper surface of the mold 50, so the composition 60 may be uniformly spread into each area of the mold 50.

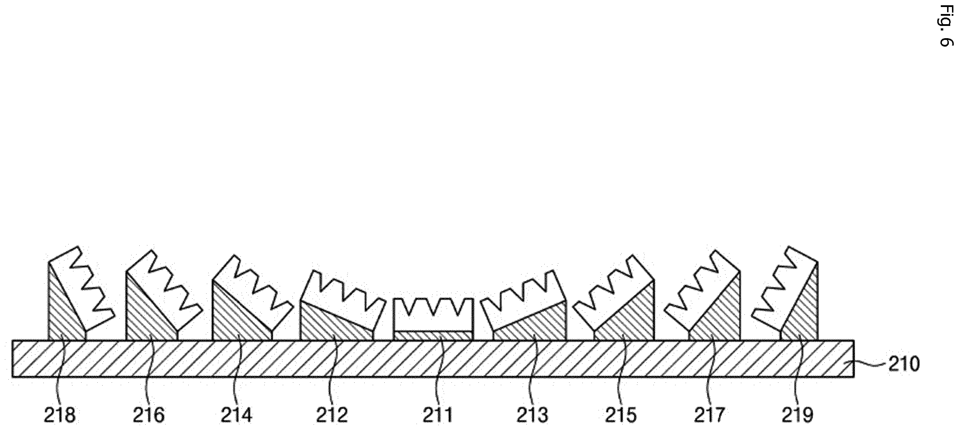

In the present exemplary embodiment, it has been described that three support regions 211, 212, and 213 are provided on the support plate 210, but the number of support regions may be changed variously. For example, referring to FIG. 6, nine support regions 211 to 219 may be provided on the support plate 210. Four support regions 212 to 219 may be provided on each side of the first support region 211 positioned at the center of the support plate 210. The upper surfaces of the support regions 211 to 219 are arranged at different angles, and the angle gradually increases as it gets farther from the center of the support plate 210. The inclination angles of the support regions 212 to 219 may be symmetrical with respect to the first support region 211.

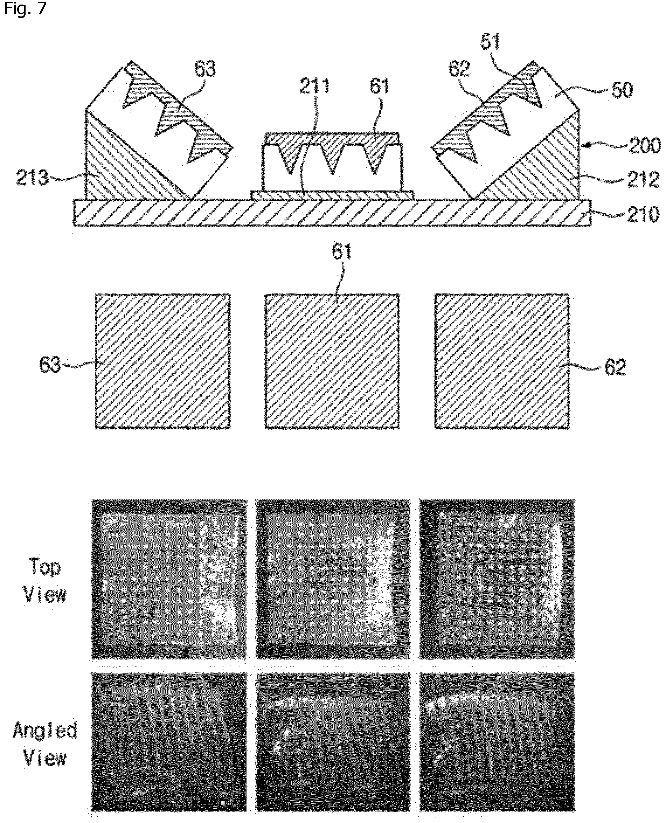

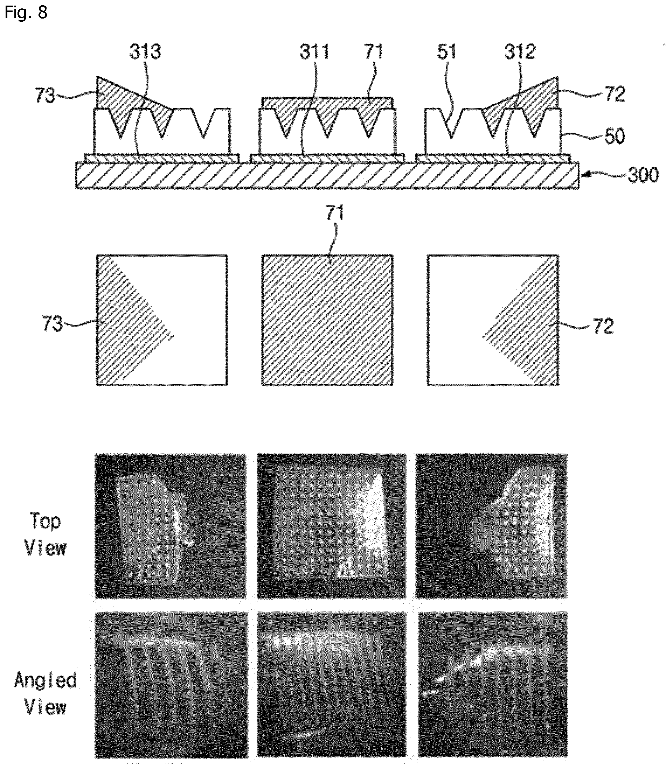

FIG. 7 is a diagram illustrating a microstructure manufactured using a mold support unit according to the exemplary embodiment of FIG. 4, and FIG. 8 is a diagram illustrating a microstructure manufactured using a mold support unit according to Comparative Embodiment. The mold support unit 300 according to Comparative Embodiment provides all upper surfaces of first to third support regions 311 to 313 as flat surfaces.

First, referring to FIG. 7, since the centrifugal force of the rotation unit 100 is provided in a direction perpendicular to the upper surfaces of the first to third support regions 211 to 213, it may be confirmed that compositions 61 to 63 are uniformly spread to each area of the mold 50 and uniformly injected into the needle groove 51.

On the other hand, referring to FIG. 8, it may be confirmed that a composition 71 is uniformly spread on the mold 50 placed on the first support region 311, but compositions 72 and 73 are concentrated on an outer region on the mold 50 placed on the second and third support regions 312 and 313. This is understood as a result of the compositions 72 and 73 being spread in the centrifugal force direction of the rotation unit 100 because the upper surfaces of the second and third support regions 312 and 313 are not disposed toward the first rotation axis 20.

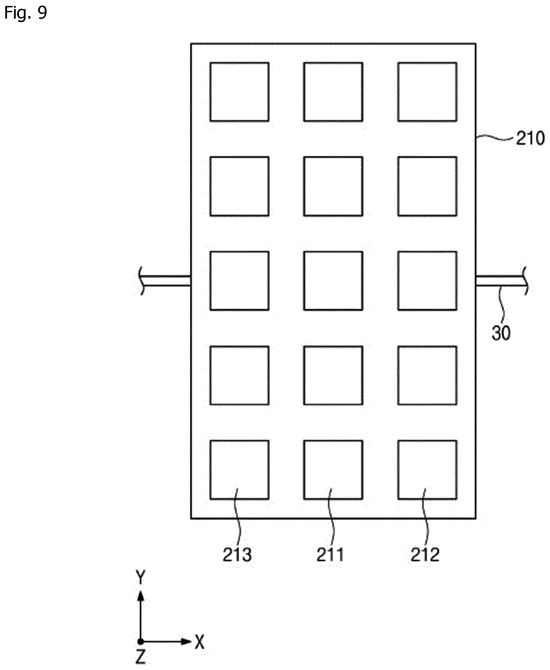

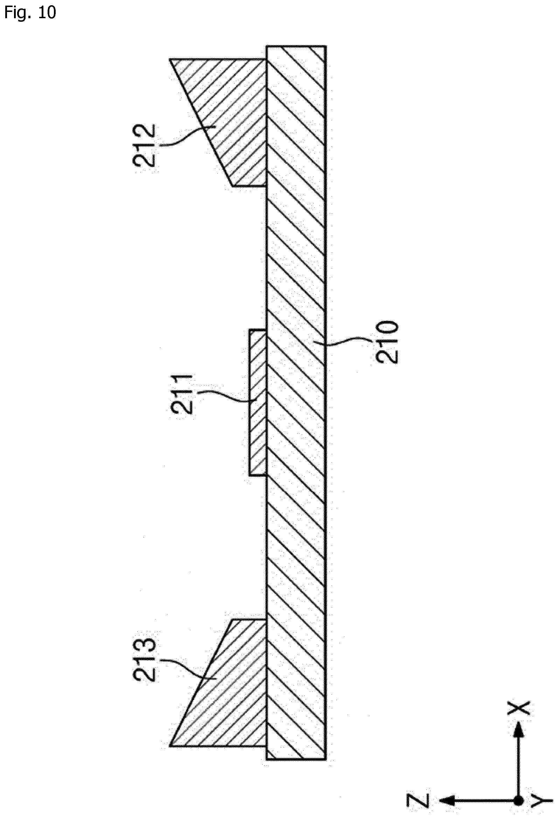

FIG. 9 is a plan view illustrating a mold support unit according to another exemplary embodiment of the present invention, and FIG. 10 is a cross-sectional view illustrating the mold support unit of FIG. 9.

Referring to FIGS. 9 and 10, the support plate 100 of the mold support unit 200 may be provided with the first to third support regions 211 to 213 described above in multiple rows. In the Y-axis direction, the first support regions 211 are arranged in a row, the second support regions 212 are arranged in a row, and the third support regions 213 are arranged in a row. The upper surfaces of the first support regions 211 are disposed as flat surfaces, the upper surfaces of the second support regions 212 are disposed to be inclined at a second angle, and the upper surfaces of the third support regions 213 are disposed to be inclined at a third angle.

When manufacturing the microstructure using the mold support unit 200 described above, a plurality of microstructures may be manufactured simultaneously in one manufacturing process.

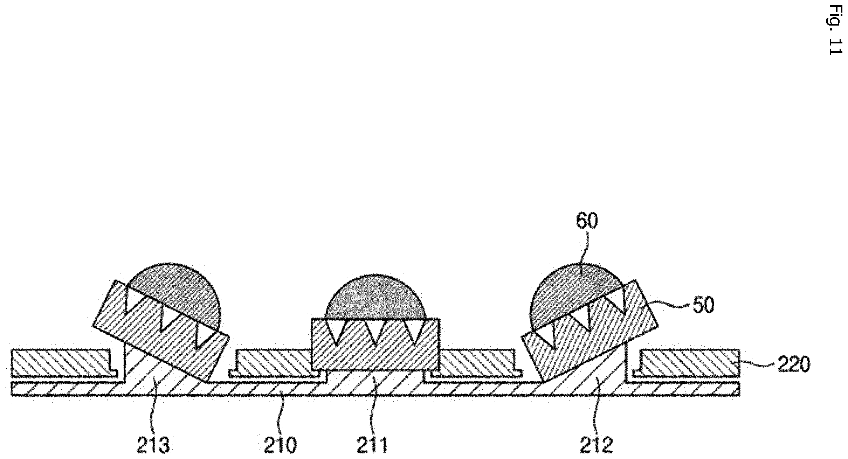

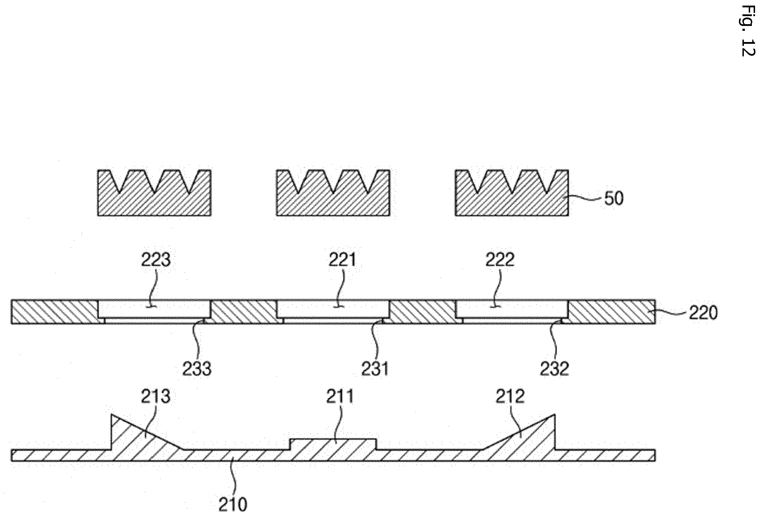

FIG. 11 is a diagram illustrating the mold support unit according to still another exemplary embodiment of the present invention, and FIG. 12 is an exploded view illustrating the mold support unit of FIG. 11.

Referring to FIGS. 11 and 12, the mold support unit 200 includes the support plate 210 and the loading plate 220.

The support plate 210 may be any one of the support plates described in FIGS. 3 to 10.

The loading plate 220 has an area corresponding to the support plate 210, and the plurality of openings 221 to 223 are formed. The openings 221 to 223 are holes penetrating through the upper surface and the lower surface of the loading plate 220, and are provided in a square shape according to an exemplary embodiment. The openings 211 are formed in a position, number, and size corresponding to the support regions 211 to 213 of the support plate 210. Support jaws 231 to 233 are formed on the inner side surface of the loading plate 220 forming the openings 221 to 223. The support jaws 231 to 233 may be formed on at least one pair of inner side surfaces facing each other among the inner side surfaces of the loading plate 220 forming the openings 221 to 223. When the mold 50 is inserted into the openings 221 to 223, the support jaw 231 to 233 support a bottom surface of the mold 50.

Hereinafter, the process of seating the mold 50 on the mold support unit 200 according to the exemplary embodiment will be described.

First, the mold 50 is positioned in the openings 221 to 223 of the loading plate 220 while the support plate 210 and the loading plate 220 are separated. Then, the loading plate 220 is moved downward while the support plate 210 and the loading plate 220 are positioned so that the support regions 211 to 213 and the openings 221 to 223 are positioned on the same line. In this process, the support regions 211 to 213 are inserted into the openings 221 to 223, and the molds 50 placed in the openings 221 to 223 are seated on the upper surfaces of the support regions 211 to 213.

As described above, the upper surfaces of some 212 and 213 of the support regions 211 to 213 are provided as inclined surfaces. It is not easy to seat the mold 50 in a certain position on the inclined surface due to the inclination. To solve this problem, the loading plate 220 is used. While the molds 50 are positioned in the openings 221 to 223 of the loading plate 220, the molds 50 are transferred to the upper surfaces of the support region 211 to 213 by coupling the loading plate 220 and the support plate 210. In this process, the molds 50 may always be seated at fixed points on the upper surfaces of the support regions 211 to 213.

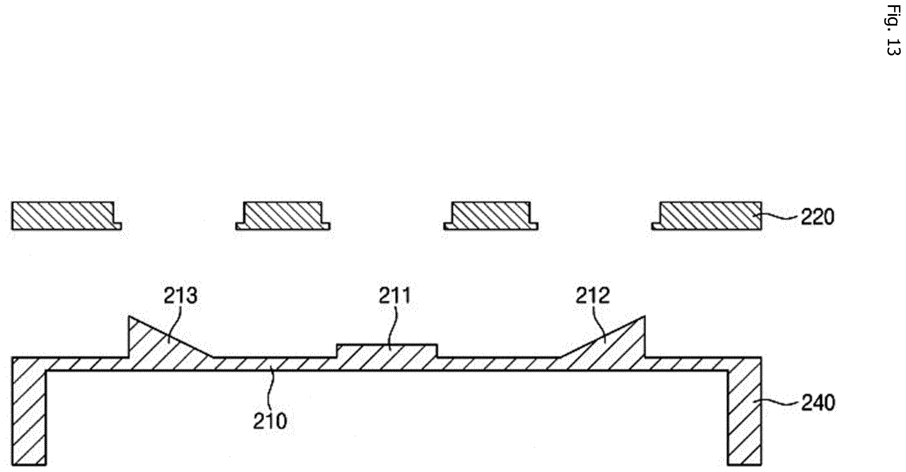

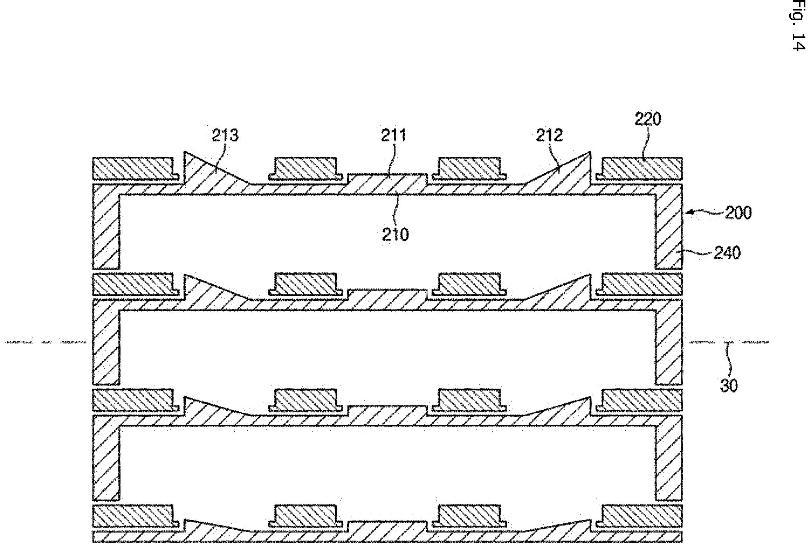

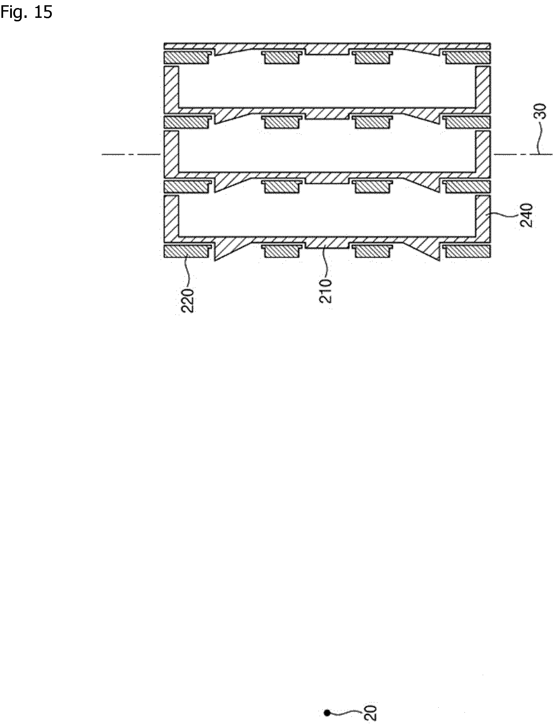

FIG. 13 is a diagram illustrating the mold support unit according to still another exemplary embodiment of the present invention, FIG. 14 is a diagram illustrating an appearance that a plurality of mold support units of FIG. 13 are stacked, and FIG. 15 is a diagram illustrating an appearance that the mold support unit of FIG. 13 rotates 90° around a second rotation axis.

Referring to FIGS. 13 and 14, a support leg 240 is formed on a bottom surface of the support plate 210. The support leg 240 is provided downward from the bottom surface of the support plate 210 to a predetermined length. A plurality of support legs 240 are provided, and are disposed to be spaced apart from each other at preset points of the support plate 210.

The plurality of mold support units 200 having the above-described structure may be stacked. Specifically, the support plate 210 included in the mold support unit 200 positioned at the upper portion thereof is placed on the upper surface of the loading plate 220 of the mold support unit 200 where the support leg 240 is positioned at the upper portion thereof. The plurality of mold support units 200 stacked in this manner are integrally coupled by a coupling means (not illustrated) and can integrally rotate around the second rotation axis 30.

All of the first support regions 211 formed on the mold support units 200 have upper surfaces arranged parallel to the upper surface of the support plate 210. The second support region 212 and the third support region 213 are provided to have different inclination angles depending on the mold support unit 200. Specifically, the inclination angles of the second support region 212 and the third support region 213 gradually decrease as the distance from the first rotation axis 20 increases. Therefore, the upper surfaces of the second support region 212 and the third support region 213 of the mold support unit 200 positioned at the lower portion thereof have a smaller inclination angle than the upper surfaces of the second support region 212 and the third support region 213 of the mold support unit 200 positioned at the upper portion thereof.

Referring to FIG. 15, when the rotation unit 100 rotates at high speed around the first rotation axis 20, the mold support units 200 rotate 90° around the second rotation axis 30 by the centrifugal force, so the support plates 210 are disposed perpendicular to the radial direction of the rotation unit 100. With the inclination angle arrangement of the upper surfaces of the first to third support regions 211, 212, and 213 described above, the upper surfaces of the first to third support regions 211, 212, and 213 may be disposed toward the first rotation axis 20.

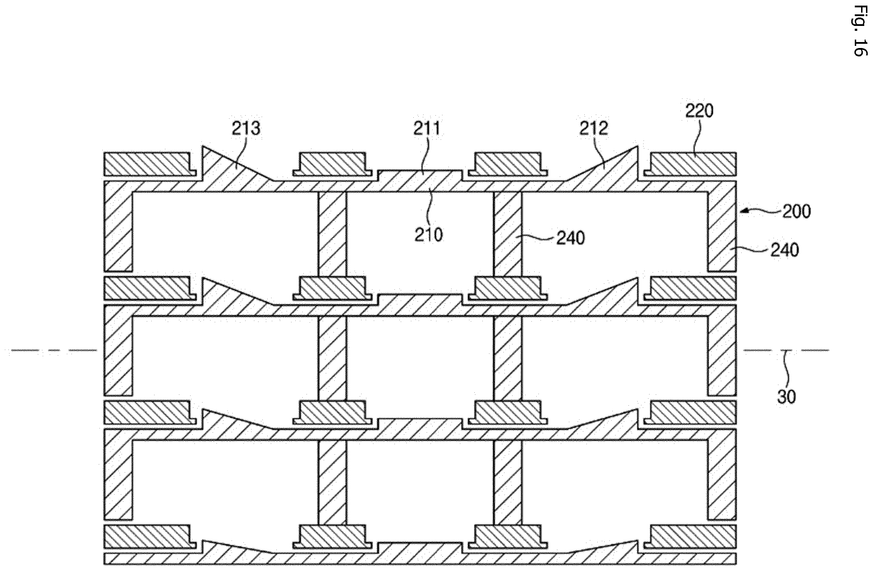

FIG. 16 is a diagram illustrating the mold support unit according to still another exemplary embodiment of the present invention.

Referring to FIG. 16, the support legs 240 may be formed not only in an edge area of a bottom surface of the support plate 210 but also in a center area thereof. The support legs 240 are placed on the upper surface of the loading plate 220 positioned at the lower portions thereof. Since the support legs 240 are disposed at regular intervals over the entire area of the support plate 210, the mold may be stably supported during high-speed rotation.

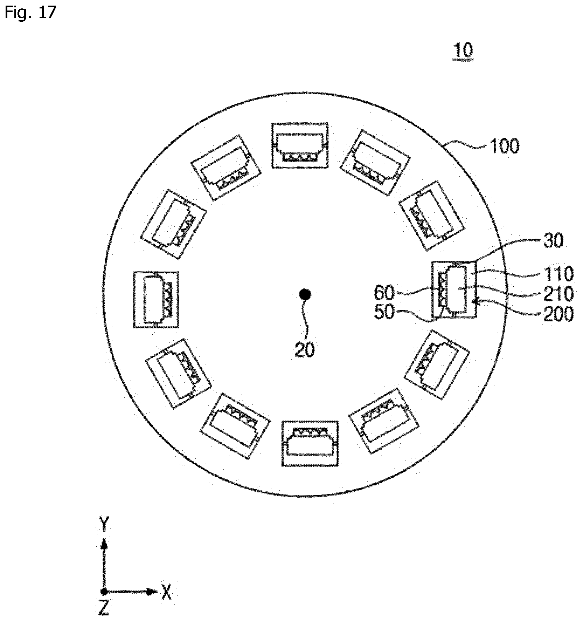

FIG. 17 is a diagram illustrating a microstructure manufacturing apparatus according to still another exemplary embodiment of the present invention.

Referring to FIG. 17, the mold support unit 200 is fixedly positioned on the second rotation axis 30 so that the upper surface of the mold 50 faces the first rotation axis 20. Therefore, while the composition 60 is supplied to the mold 50 or the rotation unit 100 rotates at high speed, the mold 50 is always positioned so that the upper surface thereof faces the first rotation axis 20. While the composition 60 is supplied to the mold 50, the composition 60 may flow down due to its own weight. Therefore, the microstructure manufacturing apparatus 10 according to the present exemplary embodiment is suitable for the composition 60 having high viscosity. Since a sufficiently large centrifugal force may be transmitted to the upper surface of the mold 50 at the high-speed rotation of the rotation unit 100, the composition 60 having high viscosity may also be spread on the upper surface of the mold 50 at a uniform thickness.

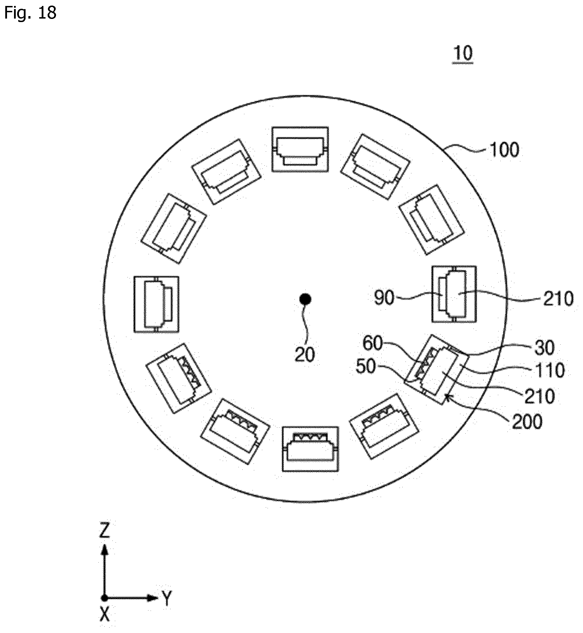

FIG. 18 is a diagram illustrating a microstructure manufacturing apparatus according to still another exemplary embodiment of the present invention.

Referring to FIG. 18, the rotation unit 100 is disposed such that the first rotation axis 20 is parallel to the ground (XY plane). Therefore, the mold support units 200 are positioned at different heights around the first rotation axis 20. Due to the structure of the rotation unit 100, the composition 60 may be supplied to the mold 50 seated on the mold support units 200 positioned at the lower portion of the first rotation axis 20. A weight forming jig 90 may be provided to other mold support units 200 to which the composition is not supplied. The weight forming jig 90 has a configuration having a weight that is the sum of the mold 50 and the composition 60, and is provided to align the center of gravity of the rotation unit 100 around the first rotation axis 20.

Hereinabove, although the present invention has been described in detail through preferred exemplary embodiments, the scope of the present invention is not limited to specific exemplary embodiments, and should be construed according to the appended claims. In addition, those skilled in the art will understand that many modifications and variations are possible without departing from the scope of the present invention.

## Claims

1. A mold support unit that supports molds for manufacturing microstructures, comprising:
a support plate in which a plurality of support regions on which the molds are placed are formed to be spaced apart from each other,
wherein the support regions include
a first support region in which an upper surface on which the mold is placed is disposed at a first angle, and
a second support region in which an upper surface on which the mold is placed is disposed at a second angle different from the first angle.

2. The mold support unit of claim 1, wherein the support regions further include a third support region whose upper surface on which the mold is placed is disposed at a third angle different from the first angle and the second angle.

3. The mold support unit of claim 2, wherein the upper surface of the first support region is disposed parallel to an upper surface of the support plate,
the upper surface of the second support region is disposed to be inclined at the second angle with respect to the upper surface of the support plate, and
the upper surface of the third support region is disposed to be inclined at the third angle with respect to the upper surface of the support plate.

4. The mold support unit of claim 3, wherein the third support region is positioned on an opposite side of the second support region with the first support region interposed therebetween, and
the upper surface of the second support region and the upper surface of the third support region are symmetrical with respect to the first support region.

5. The mold support unit of claim 2, wherein the first support region to the third support region are sequentially positioned in one direction, and
the third angle with respect to an upper surface of the support plate is larger than the second angle.

6. The mold support unit of claim 1, further comprising:
a loading plate that is provided with a plurality of openings formed such that the support regions are individually inserted inward and has a support jaw formed on an inner side surface that defines the opening and on which the mold is placed.

7. The mold support unit of claim 6, wherein the support regions and the openings correspond one-to-one.

8. The mold support unit of claim 1, wherein a plurality of support legs are formed on a bottom surface of the support plate and extend downward to a predetermined length.

9. The mold support unit of claim 8, wherein the plurality of support plates are stacked in a vertical direction, and the support legs are placed on an upper surface of the support plate positioned at a lower portion thereof.

10. A microstructure manufacturing apparatus, comprising:
a rotation unit rotatable around a first rotation axis; and
a mold support unit coupled to the rotation unit at a preset distance from the first rotation axis, capable of relative rotation around the rotation unit around a second rotation axis, and supporting a mold for manufacturing a microstructure,
wherein the mold support unit includes a support plate having a support region formed on which the mold is placed, and
the mold support unit rotates around the second rotation axis by centrifugal force of the rotation unit rotating around the first rotation axis so that the support region faces the first rotation axis.

11. The microstructure manufacturing apparatus of claim 10, wherein the plurality of mold support units are disposed in a ring shape based on the first rotation axis and are positioned at the same distance from the first rotation axis.

12. The microstructure manufacturing apparatus of claim 10, wherein the support region includes:
a first support region in which an upper surface on which the mold is placed is disposed at a first angle; and
a second support region in which an upper surface on which the mold is placed is disposed at a second angle different from the first angle.

13. The microstructure manufacturing apparatus of claim 12, wherein the upper surface of the first support region is disposed parallel to an upper surface of the support plate, and
the upper surface of the second support region is disposed to be inclined at the second angle with respect to the upper surface of the support plate.

14. The microstructure manufacturing apparatus of claim 12, wherein the upper surface of the first support region is disposed parallel to an upper surface of the support plate, and
the upper surface of the second support region is disposed to be inclined with respect to the upper surface of the support plate.

15. The microstructure manufacturing apparatus of claim 10, wherein the mold support unit further includes a loading plate that is provided with an opening formed such that the support region is inserted inward and has a support jaw formed on an inner side surface that defines the opening and on which the mold is placed.
